# EUROPEAN PATENT APPLICATION

(11) **EP 4 772 176 A1**
(43) Date of publication of application: **08.07.2026**
(21) Application number: 24884809.5
(22) Date of filing: 30.10.2024
(51) Int. Cl.: A61K 31/05, A61P 17/00, A61P 17/06, A61P 17/08, A61K 9/06

(54) **PHARMACEUTICAL COMPOSITION OF PHENOL DERIVATIVE HAVING IMPROVED STABILITY AND USE THEREOF**

(30) Priority: 02.11.2023 CN 202311442719
(71) Applicant: Thederma Shanghai Co., Ltd., Shanghai 201203 (CN)
(72) Inventor: JIA, Jianmin, Shanghai 201203 (CN); MIAO, Pengfei, Shanghai 201203 (CN); CHEN, Ming, Shanghai 201203 (CN); MA, Zhilong, Shanghai 201203 (CN)
(74) Representative: Ipsilon
(86) International application number: PCT/CN2024/128467
(87) International publication number: WO 2025/092809

(57) **Abstract**

A pharmaceutical composition of a phenol derivative and use thereof. The pharmaceutical composition comprises the following components: an active ingredient, a thickening agent, an emulsifier, a humectant, a chelating agent, a pH regulator, an oil phase matrix, and water, wherein the active ingredient is selected from at least one of a compound of formula (I) and a pharmaceutically acceptable salt thereof; the active ingredient is dispersed in a homogeneous and uniform emulsion drop form and has good physical and chemical stability. The composition is suitable for the treatment of atopic dermatitis, psoriasis and seborrheic dermatitis, especially for the treatment of atopic dermatitis and seborrheic dermatitis of children, and has good safety, stability, and effectiveness, and uniform morphology characteristics.

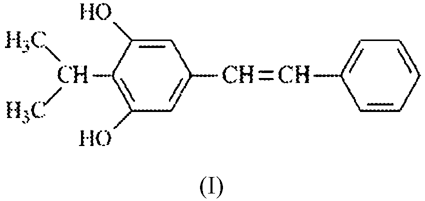

## Description

This application claims priority to Chinese Patent Application No. 202311442719.6 entitled "PHARMACEUTICAL COMPOSITION OF PHENOL DERIVATIVE HAVING IMPROVED STABILITY AND USE THEREOF" filed with CNIPA on November 2, 2023, the disclosure of which is incorporated herein by reference in its entirety.

### TECHNICAL FIELD

The present disclosure belongs to the field of pharmaceutical compositions, and specifically relates to a pharmaceutical composition of a phenol derivative with improved stability and use thereof.

### BACKGROUND

Atopic dermatitis (AD) is a chronic, recurrent, inflammatory skin condition. In recent years, the incidence rate of AD has been on the rise worldwide. The etiology and pathogenesis of AD are still unclear and may be related to a variety of factors, including genetics, skin barrier defects, immunity, environment, and microbial colonization. At present, the treatment of mild to moderate childhood AD is usually based on external glucocorticoids and calcineurin inhibitors, while the treatment of moderate, severe or refractory childhood AD is difficult and requires individualized treatment, and there is no systematic and universal drug treatment, which seriously affects the life quality and mental health of children.

Seborrheic dermatitis (SD) is a chronic papulosquamous, superficial inflammatory skin disease that occurs at the site of seborrhea, typically the dark red or yellow-red spots formed by the fusion of rash follicular papules, covered with greasy scales or crusts, and may be accompanied by varying degrees of pruritus. SD is a chronic, recurrent inflammatory skin condition that typically presents as a red scaly rash. The incidence in the healthy population is 5%, with 70.3% of cases occurring on the head. It is common in infants, adolescents, and adults. Seborrheic dermatitis refers to a scalp disease, differing from simple dandruff in that it presents with erythema as a sign of inflammation, exhibits greater scalp desquamation, and is sometimes accompanied by itching and burning sensations, along with eczematous lesions on other body areas. The disease can occur in the form of patches, and also often affects the entire scalp, extending beyond the hairline to often include the forehead, around the neck, and both ears. In severe cases, the scalp may develop secondary infections, with changes potentially manifesting as a spongy texture, formation of vesicles and crusts, and possible exudation. Seborrheic dermatitis also frequently occurs in infancy, typically resolving spontaneously between 8-12 months of age. Scalp changes in infants include erythema, flaking scalp, and occasional blisters and crusts that resolve spontaneously over a few weeks, recur intermittently, or persist throughout childhood. These changes are usually accompanied by similar lesions around the eyelids, nose, and ears. In addition, the condition occurs frequently after puberty and can last a lifetime, and can even be more severe. About 1-3% of the population suffers from the disease.

The etiology and pathogenesis of SD are still unclear, and the complex interaction of *Malassezia,* keratinocytes and immune response plays a crucial role in the pathogenesis of SD. The inflammatory skin diseases, such as seborrheic dermatitis and atopic dermatitis, share some of the causative factors, including regulation of factors that stimulate keratinocyte proliferation and differentiation, and disruption of the skin barrier. Atopic dermatitis typically presents with a Th2 immune response with elevated levels of interleukin (IL)-4 and IL-13; and seborrheic dermatitis is mainly a reaction of Th2 and Th17, with elevated levels of IL-4, IL-17, and IL-8.

Currently, the treatment methods for seborrheic dermatitis include simple anti-inflammatory drugs (such as hormone external preparations and calcineurin inhibitors), supplemented with skin moisturizing products, which have a relatively low efficacy; and symptoms are prone to recurrence after stopping the medication.

Although CN108066279A and CN113797159A disclose research on the external cream of Benvitimod, the provided sample is poor in characteristics and still contains a significant proportion of block-like oily substances, affecting the performance of the product. Moreover, the embodiment of CN114042041A indicates that the white petroleum jelly in the formulation leads to the generation of waxy substances; even the formulation containing a lower level of white petroleum jelly results in an uneven emulsion, while the formulation without petroleum jelly has a uniform emulsion.

Therefore, it is an urgent technical problem in the art to provide a pharmaceutical composition/preparation for the treatment of childhood AD and a pharmaceutical composition/preparation for the treatment of SD that can eliminate the anxiety and fear caused by hormone medications in children, while ensuring safety, stability, effectiveness, and uniform morphological characteristics.

### SUMMARY

To solve the above technical problem, the present disclosure provides a pharmaceutical composition, which includes the following components: an active ingredient, a thickening agent, an emulsifier, a humectant, a chelating agent, a pH-adjusting agent, an oil phase matrix, and water, wherein the active ingredient is selected from at least one of the compound shown in Formula (I) and pharmaceutically acceptable salts thereof;

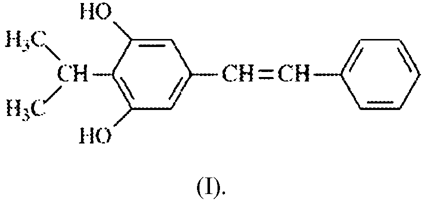

According to an embodiment of the present disclosure, the compound shown in Formula (I) may be either an E-isomer or a Z-isomer, preferably the E-isomer shown in Formula (I-1).

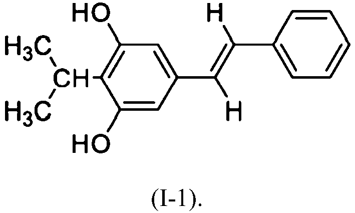

According to a preferred embodiment of the present disclosure, the compound shown in Formula (I-1) is Benvitimod.

According to an embodiment of the present disclosure, the pharmaceutically acceptable salt may be selected from an additive salt of the compound in Formula (I) and an alkali.

In a preferred embodiment, the pharmaceutical composition uses only the compound shown in Formula (I) as the active ingredient, and preferably only the E-isomer shown in Formula (I-1) (i.e., Benvitimod) as the active ingredient.

According to an embodiment of the present disclosure, in the pharmaceutical composition, the active ingredient exists in the form of emulsion droplets, preferably a diameter of the emulsion droplet is less than 10 µm, for example, less than 5 µm.

According to an embodiment of the present disclosure, in the pharmaceutical composition, the active ingredient is 0.1-8 parts by weight, for example, 0.5-5 parts, and for example, 0.5 parts, 0.75 parts, 1 part, 1.25 parts, 1.5 parts, 2 parts, 2.5 parts, 3 parts, 3.5 parts, or 4 parts.

According to an embodiment of the present disclosure, the thickening agent is selected from one or more of cetyl alcohol, stearyl alcohol, and cetostearyl alcohol.

According to an embodiment of the present disclosure, in the pharmaceutical composition, the thickening agent is 2-15 parts by weight, for example, 5-10 parts by weight, and for example, 6 parts, 7 parts, or 8 parts.

According to an embodiment of the present disclosure, the emulsifier is selected from one or more of polyethylene glycol hexadecyl octadecyl ether, glyceryl mono and distearate, polyethylene glycol stearate (such as pegoxol-7 stearate), polyoxyethylene hydrogenated castor oil (polyoxyethylene (54) hydrogenated castor oil), and polysorbate 80. In some embodiments, the emulsifier is polyethylene glycol hexadecyl octadecyl ether, glyceryl mono and distearate, and polysorbate 80; in other embodiments, the emulsifier is polyethylene glycol hexadecyl octadecyl ether and glyceryl mono and distearate; and in other embodiments, the emulsifier is pegoxol-7 stearate and polysorbate 80. Preferably, the emulsifier includes at least polyethylene glycol hexadecyl octadecyl ether and glyceryl mono and distearate, for example, the emulsifier is composed of polyethylene glycol hexadecyl octadecyl ether and glyceryl mono and distearate, or composed of polyethylene glycol hexadecyl octadecyl ether, glyceryl mono and distearate, and polysorbate 80.

According to an embodiment of the present disclosure, in the pharmaceutical composition, the emulsifier is 2-25 parts by weight, for example, 5-20 parts, and for example, 6 parts, 6.5 parts, 7 parts, 7.5 parts, 8 parts, 10 parts, 12 parts, or 16 parts.

According to an embodiment of the present disclosure, in a case where the pharmaceutical composition includes two types of emulsifiers, a mass ratio of the two types of emulsifiers may be (1-3):(3-1), for example, (1-2):(2-1), (1-1.5):(1.5-1), or (1-1.2):(1.2-1), and a specific example may be 3:3.5, 3.5:3, or 1:1. Illustratively, if the emulsifier is polyethylene glycol hexadecyl octadecyl ether and glyceryl mono and distearate, a mass ratio of polyethylene glycol hexadecyl octadecyl ether to glyceryl mono and distearate may be (1-3):(3-1), (1-2):(2-1), (1-1.5):(1.5-1), or (1-1.2):(1.2-1), and a specific example may be 3:3.5, 3.5:3, or 1:1.

According to an embodiment of the present disclosure, in a case where the pharmaceutical composition includes three types of emulsifiers, based on the total weight of the emulsifiers, a mass fraction of each emulsifier of the total mass of the emulsifiers may be independently selected from 1/5-1/2, for example, 1/4-1/3, or 2/7-1/3.

In some embodiments, if there are three types of emulsifiers, a mass ratio of the three emulsifiers may be (1.5-3):(1.5-3):1, for example, (1.7-2.5):(1.7-2.5):1, and for example, 3.5:3:1.5. For example, the emulsifier is composed of polyethylene glycol hexadecyl octadecyl ether, glyceryl mono and distearate, and polysorbate 80, and a mass ratio of polyethylene glycol hexadecyl octadecyl ether, glyceryl mono and distearate, and polysorbate 80 may be (1.5-3):(1.5-3):1, for example, (1.7-2.5):(1.7-2.5):1, and for example, 3.5:3:1.5.

According to an embodiment of the present disclosure, the humectant is selected from one or more of propylene glycol, glycerol, and polyethylene glycol, and preferably, propylene glycol.

According to an embodiment of the present disclosure, in the pharmaceutical composition, the humectant is 3-15 parts by weight, for example, 5-12 parts, and for example, 6 parts, 7 parts, 8 parts, 9 parts, or 10 parts.

According to an embodiment of the present disclosure, the chelating agent is selected from a metal-ion chelating agent, for example, selected from one or more of ethylenediaminetetraacetic acid, sodium gluconate, nitrilotriacetic acid, tartaric acid, citric acid and salts thereof; preferably, the salt is a sodium salt, a potassium salt, or an ammonium salt; in some embodiments, the chelating agent is selected from disodium ethylenediaminetetraacetate (also known as disodium edetate).

According to an embodiment of the present disclosure, in the pharmaceutical composition, the chelating agent is 0.01-1 part by weight, for example, 0.05-0.5 parts, and for example, 0.05 parts, 0.1 parts, 0.2 parts, or 0.5 parts.

According to an embodiment of the present disclosure, the pH-adjusting agent is selected from a pH buffer solution, and preferably a pH buffer solution of an organic acid and/or a salt thereof; for example, the pH-adjusting agent is selected from one or more of a citric acid/sodium citrate buffer solution, a tartrate buffer solution, a malic acid buffer solution, an acetic acid/sodium acetate buffer solution, and preferably the citric acid/sodium citrate buffer solution.

According to an embodiment of the present disclosure, the oil phase matrix may include one or more of petrolatum, medium-chain triglycerides, and paraffin, for example, the oil phase matrix includes one or two of petrolatum and medium-chain triglycerides. The petrolatum is preferably white petrolatum. Preferably, the oil phase matrix includes petrolatum and optionally existing or non-existing one or two of medium-chain triglycerides and paraffin; more preferably, the oil phase matrix is petrolatum and medium-chain triglycerides.

According to an embodiment of the present disclosure, in the pharmaceutical composition, the oil phase matrix is totally 5-25 parts by weight, for example, 8-20 parts, and for example, 9 parts, 10 parts, 12 parts, 13 parts, 14 parts, 15 parts, 16 parts, or 17 parts.

According to an embodiment of the present disclosure, in the pharmaceutical composition, the petrolatum (preferably white petrolatum) is 1-9 parts by weight, for example, 1-8 parts, 1-7 parts, 1-6 parts, 1-5 parts, 2-6 parts, 3-6 parts, or 4-6 parts, and for example, 1 part, 1.5 parts, 2 parts, 2.5 parts, 3 parts, 3.5 parts, 4 parts, 4.5 parts, 5 parts, 5.5 parts, or 6 parts, or more than 1 part, more than 1.5 parts, more than 2 parts, more than 2.5 parts, more than 3 parts, more than 3.5 parts, more than 4 parts, more than 4.5 parts, more than 5 parts, more than 5.5 parts, or more than 6 parts.

According to an embodiment of the present disclosure, the water is preferably purified water.

According to an embodiment of the present disclosure, in the pharmaceutical composition, the water is 40-70 parts by weight, for example, 45-60 parts, or 50-65 parts, and for example, 45 parts, 46 parts, 47 parts, 48 parts, 49 parts, 50 parts, 55 parts, 56 parts, 57 parts, 58 parts, 59 parts, 60 parts, 61 parts, 62 parts, 63 parts, 64 parts, or 65 parts.

According to an embodiment of the present disclosure, the pharmaceutical composition further includes an antioxidant, a preservative, and/or a penetration enhancer.

According to an embodiment of the present disclosure, the antioxidant is selected from one or more of propyl gallate and butylated hydroxytoluene (BHT); preferably, the antioxidant is butylated hydroxytoluene.

According to an embodiment of the present disclosure, in the pharmaceutical composition, the antioxidant is 0.01-0.2 parts by weight, for example, 0.05-0.15 parts, and for example, 0.05 parts, 0.1 parts, or 0.12 parts.

According to an embodiment of the present disclosure, the preservative is selected from one or more of methyl hydroxybenzoate, ethyl hydroxybenzoate, and propyl hydroxybenzoate, and preferably, ethyl hydroxybenzoate.

According to an embodiment of the present disclosure, in the pharmaceutical composition, the preservative is 0.01-0.3 parts by weight, for example, 0.05-0.15 parts, and for example, 0.1 parts, 0.1 parts, 0.15 parts, or 0.2 parts.

According to an embodiment of the present disclosure, the penetration enhancer is selected from one or more of diethylene glycol monoethyl ether, isopropyl myristate, isopropyl palmitate, menthol, caprylocaproyl polyoxylglycerides, N-methyl pyrrolidone, propylene carbonate, and isosorbide dimethyl ether, and preferably diethylene glycol monoethyl ether.

According to an embodiment of the present disclosure, in the pharmaceutical composition, the penetration enhancer is 0.1-5 parts by weight, for example, 0.5-3 parts, and for example, 1 part, 2 parts, 3 parts, or 4 parts.

According to an embodiment of the present disclosure, the pharmaceutical composition has a pH of 4-6.5, for example, 4.5, 5, 5.2, 5.5, or 6.

In some embodiments, the humectant and the penetration enhancer additionally serve as solvents during oil-phase formulation.

According to an embodiment of the present disclosure, the pharmaceutical composition includes the following components in parts by weight: 0.1-8 parts of the active ingredient, 2-15 parts of the thickening agent, 2-25 parts of the emulsifier, 3-15 parts of the humectant, 0.01-1 part of the chelating agent, 0.01-0.2 parts of the antioxidant, 0.01-0.3 parts of the preservative, 0-5 parts of the penetration enhancer, 5-25 parts of the oil phase matrix, 40-65 parts of water, and an appropriate amount of the pH-adjusting agent;
the active ingredient is the compound shown in Formula (I), and preferably, the E-isomer shown in Formula (I-1) as the active ingredient;
preferably, the thickening agent is stearyl alcohol or cetostearyl alcohol;
preferably, the emulsifier is selected from any one of the following combinations: polyethylene glycol hexadecyl octadecyl ether, glyceryl mono and distearate, and polysorbate 80; polyethylene glycol hexadecyl octadecyl ether, and glyceryl mono and distearate; or pegoxol-7 stearate and polysorbate 80;
preferably, the humectant is selected from propylene glycol;
preferably, the chelating agent is selected from disodium ethylenediaminetetraacetate;
preferably, the oil phase matrix is selected from one, two, or three of petrolatum, medium-chain triglycerides, and paraffin, and preferably white petrolatum and medium-chain triglycerides;
preferably, the pH-adjusting agent is selected from a citric acid/sodium citrate buffer solution or an acetic acid/sodium acetate buffer solution;
preferably, the antioxidant is selected from butylated hydroxytoluene;
preferably, the preservative is ethyl hydroxybenzoate;
preferably, the penetration enhancer is diethylene glycol monoethyl ether;
preferably, the pharmaceutical composition has a pH of 4-6.5.

Preferably, in the embodiment of each pharmaceutical composition, the sum of the parts by weight of each component in the pharmaceutical composition is 100 parts.

In some embodiments, the pharmaceutical composition includes the following components in parts by weight:
0.5-5 parts of the E-isomer of the compound shown in Formula (I), 5-10 parts of cetostearyl alcohol, 5-20 parts of the emulsifier, 5-12 parts of propylene glycol, 0.05-0.5 parts of the chelating agent, 0.01-0.2 parts of the antioxidant, 0.01-0.3 parts of the preservative, 0-5 parts of the penetration enhancer, 8-20 parts of the oil phase matrix, 40-65 parts of the water, and an appropriate amount of the pH-adjusting agent;
preferably, the emulsifier is composed of polyethylene glycol hexadecyl octadecyl ether, glyceryl mono and distearate, and polysorbate 80 in a mass ratio of (1.5-3):(1.5-3):1;
preferably, the oil phase matrix is composed of white petrolatum and medium-chain triglycerides, wherein the white petrolatum is 1-9 parts.

In some embodiments, the pharmaceutical composition is selected from any one of the formulations with components of the following parts by weight:
Formulation1: 1 part of E-isomer of the compound shown in Formula (I), 2 parts of white petrolatum; 7 parts of cetostearyl alcohol, 3.5 parts of polyethylene glycol hexadecyl octadecyl ether, 10 parts of medium-chain triglycerides, 3 parts of glyceryl mono and distearate, 0.1 parts of butylated hydroxytoluene, 0.15 parts of ethyl hydroxybenzoate, 1.5 parts of polysorbate 80, 10 parts of propylene glycol, 0.05 parts of disodium edetate, 61.7 parts of water, and an appropriate amount of citric acid/sodium citrate;
Formulation 2: 1 part of E-isomer of the compound shown in Formula (I), 2 parts of white petrolatum; 7 parts of cetostearyl alcohol, 3.5 parts of polyethylene glycol hexadecyl octadecyl ether, 10 parts of medium-chain triglycerides, 3 parts of glyceryl mono and distearate, 0.1 parts of butylated hydroxytoluene, 0.15 parts of ethyl hydroxybenzoate, 1.5 parts of polysorbate 80, 2 parts of diethylene glycol monoethyl ether, 8 parts of propyl glycol, 0.05 parts of disodium edetate, 61.7 parts of water, and an appropriate amount of citric acid/sodium citrate;
Formulation 3: 4 parts of E-isomer of the compound shown in Formula (I), 2 parts of white petrolatum; 7 parts of cetostearyl alcohol, 3.5 parts of polyethylene glycol hexadecyl octadecyl ether, 10 parts of medium-chain triglycerides, 3 parts of glyceryl mono and distearate, 0.1 parts of butylated hydroxytoluene, 0.15 parts of ethyl hydroxybenzoate, 1.5 parts of polysorbate 80, 2 parts of diethylene glycol monoethyl ether, 8 parts of propyl glycol, 0.05 parts of disodium edetate, 58.7 parts of water, and an appropriate amount of citric acid/sodium citrate;
Formulation 4: 0.5 parts of E-isomer of the compound shown in Formula (I), 2 parts of white petrolatum; 7 parts of cetostearyl alcohol, 3.5 parts of polyethylene glycol hexadecyl octadecyl ether, 10 parts of medium-chain triglycerides, 3 parts of glyceryl mono and distearate, 0.1 parts of butylated hydroxytoluene, 0.15 parts of ethyl hydroxybenzoate, 1.5 parts of polysorbate 80, 2 parts of diethylene glycol monoethyl ether, 8 parts of propyl glycol, 0.05 parts of disodium edetate, 62.2 parts of water, and an appropriate amount of citric acid/sodium citrate;
Formulation 5: 2 parts of E-isomer of the compound shown in Formula (I), 2 parts of white petrolatum; 7 parts of cetostearyl alcohol, 3.5 parts of polyethylene glycol hexadecyl octadecyl ether, 10 parts of medium-chain triglycerides, 3 parts of glyceryl mono and distearate, 0.1 parts of butylated hydroxytoluene, 0.15 parts of ethyl hydroxybenzoate, 1.5 parts of polysorbate 80, 2 parts of diethylene glycol monoethyl ether, 8 parts of propyl glycol, 0.05 parts of disodium edetate, 60.7 parts of water, and an appropriate amount of citric acid/sodium citrate;
Formulation 6: 3 parts of E-isomer of the compound shown in Formula (I), 2 parts of white petrolatum; 7 parts of cetostearyl alcohol, 3.5 parts of polyethylene glycol hexadecyl octadecyl ether, 10 parts of medium-chain triglycerides, 3 parts of glyceryl mono and distearate, 0.1 parts of butylated hydroxytoluene, 0.15 parts of ethyl hydroxybenzoate, 1.5 parts of polysorbate 80, 2 parts of diethylene glycol monoethyl ether, 8 parts of propyl glycol, 0.05 parts of disodium edetate, 59.7 parts of water, and an appropriate amount of citric acid/sodium citrate.

In some embodiments, the pharmaceutical composition may further include a second active ingredient.

The present disclosure further provides a preparation method for the above pharmaceutical composition, including mixing the components to obtain the pharmaceutical composition.

In some embodiments, the preparation method includes the following steps:
(A1) mixing a chelating agent, a pH-adjusting agent, and water, followed by heating to obtain an aqueous phase solution;
(A2) mixing an oil phase matrix, an emulsifier, a thickening agent, and optionally containing or not containing a preservative and an antioxidant, followed by heating to dissolve, to obtain an oil phase solution;
(A3) mixing an active ingredient, a humectant, and optionally containing or not containing a penetration enhancer, followed by heating to dissolve, to obtain a third phase solution;
(A4) transferring the aqueous phase solution to the oil phase solution, emulsifying, and cooling; and
(A5) adding the third phase solution to the emulsion system in step (A4), emulsifying, and cooling to obtain the pharmaceutical composition.

According to an embodiment of the present disclosure, a pH of the aqueous phase solution is 4-6.5, for example, 5-6.

According to an embodiment of the present disclosure, in step (A1) or step (A2), the heating is performed at a temperature of 60-90°C, for example, 70-80°C.

According to an embodiment of the present disclosure, in step (A3), the heating is performed at a temperature of 50-65°C, for example, 55-60°C.

According to an embodiment of the present disclosure, in step (A4), the cooling is performed until the temperature reaches 50-65°C, for example, 55-60°C.

According to an embodiment of the present disclosure, in step (A4) or step (A5), the emulsifying is performed for a period of 10-25 min, for example, 15 min, or 20 min.

In an embodiment, the preparation method includes the following steps:
(B1) mixing a chelating agent, a pH-adjusting agent, and water, followed by heating to obtain an aqueous phase solution;
(B2) mixing an oil phase matrix, an emulsifier, a thickening agent, and optionally containing or not containing a preservative and an antioxidant, followed by heating to dissolve, to obtain an oil phase solution;
(B3) mixing an active ingredient, a humectant, and optionally containing or not containing a penetration enhancer, followed by heating to dissolve, to obtain a third phase solution;
(B4) transferring the third phase solution to the oil phase solution, and performing heat preservation and stirring; and
(B5) adding the aqueous phase solution to the mixed system in step (B4), emulsifying, and cooling, and re-emulsifying, and re-cooling, to obtain the pharmaceutical composition.

According to an embodiment of the present disclosure, a pH of the aqueous phase solution is 4-6.5, for example, 5-6.

According to an embodiment of the present disclosure, in step (B1) or step (B2), the heating is performed at a temperature of 60-90°C, for example, 70-80°C.

According to an embodiment of the present disclosure, in step (B3), the heating is performed at a temperature of 50-65°C, for example, 55-60°C.

According to an embodiment of the present disclosure, in step (B5), the duration of the first emulsification is longer than that of the second emulsification; for example, the first emulsifying is performed for a period of 10-25 min, for example, 15 min, or 20 min.

According to an embodiment of the present disclosure, in step (B5), the first cooling is to reduce the temperature to 48-52°C.

In an embodiment, the preparation method includes the following steps:
(C1) mixing a chelating agent, a pH-adjusting agent, and water, followed by heating to obtain an aqueous phase solution;
(C2) mixing an active ingredient, an oil phase matrix, an emulsifier, a thickening agent, a humectant, and optionally containing or not containing a preservative, an antioxidant, and a penetration enhancer, followed by heating to dissolve, to obtain an oil phase solution; and
(C3) transferring the oil phase solution to the aqueous phase solution, emulsifying, and cooling, to obtain the pharmaceutical composition.

According to an embodiment of the present disclosure, a pH of the aqueous phase solution is 4-6.5.

According to an embodiment of the present disclosure, in step (C1) or step (C2), the heating is performed at a temperature of 60-90°C, for example, 70-80°C.

According to an embodiment of the present disclosure, in step (C3), the emulsification is performed for a period of 5-20 min, for example, 10 min, or 15 min.

The present disclosure further provides use of the above pharmaceutical composition in the preparation of a pharmaceutical preparation, and preferably, the pharmaceutical preparation is a cream preparation.

The present disclosure further provides a pharmaceutical preparation, and preferably a cream preparation, including the above pharmaceutical composition.

According to an embodiment of the present disclosure, in the pharmaceutical preparation, a mass percentage of the active ingredient (i.e., at least one of the compound shown in Formula (I) and pharmaceutically acceptable salts thereof) is 0.1-8%, such as 0.5-5%, and for example, 0.5%, 0.75%, 1%, 1.25%, 1.5%, 1.75%, 2%, 2.5%, 3%, 3.5%, or 4%.

According to an embodiment of the present disclosure, the pharmaceutical composition or the pharmaceutical preparation is used for treating atopic dermatitis and psoriasis, wherein the atopic dermatitis is preferably pediatric atopic dermatitis, for example, mild, moderate, severe, or refractory pediatric atopic dermatitis.

In another aspect, the present disclosure relates to use of the above pharmaceutical composition in the preparation of drugs for treating and/or preventing atopic dermatitis and psoriasis; wherein the atopic dermatitis is preferably pediatric atopic dermatitis, and further preferably, mild, moderate, severe, or refractory pediatric atopic dermatitis.

In another aspect, the present disclosure relates to use of the above pharmaceutical composition in the preparation of drugs for treating and/or preventing seborrheic dermatitis; wherein the seborrheic dermatitis is preferably sebopsoriasis.

In an embodiment, an administration route of the pharmaceutical composition or pharmaceutical preparation is topical administration, or further in combination with oral administration preparations or other topical administration preparations.

The present disclosure further provides a method for treating and/or preventing atopic dermatitis, psoriasis, and/or seborrheic dermatitis, including administering an effective amount or therapeutic effective amount of the pharmaceutical composition or the pharmaceutical preparation to a patient; wherein the atopic dermatitis is preferably pediatric atopic dermatitis, and the seborrheic dermatitis is preferably sebopsoriasis.

### Definition and explanation of terms

Unless otherwise specified, the term definitions contained in the specifications and claims of the present application, including definition as an example, exemplary definition, preferred definitions, definitions recorded in tables, and definitions of specific compounds in embodiments, and may be combined and integrated with each other arbitrarily. Such combinations and integrations should fall within the recording scope of the specifications in the present application.

The term "effective amount" or "therapeutically effective amount" refers to the amount of the compound in the present disclosure that is sufficient to achieve the intended application (including but not limited to the treatment of the diseases defined below). The therapeutically effective amount may vary depending on factors such as the intended application (*in vitro* or *in vivo*)*,* the subject being treated, and the characteristics of the disease, such as the subject's weight and age, the severity of the disease, and the route of administration, which can be readily determined by a person skilled in the art. The specific dosage will vary depending on factors such as the selected specific compound, the administration plan upon which it is based, whether it is administered in combination with other compounds, the timing of administration, the tissues to which it is administered, and the physical delivery system employed.

The term "patient" refers to individuals who need preventive measures or treatment for diseases related to anaerobic bacteria, wherein the patient is a mammal, such as a rodent, cattle, a pig, a dog, a cat, and a primate, especially a human.

The term "children" refers to patients aged over 3 months, for example, patients from 3 months - 18 years, preferably patients from 3 months - 14 years.

### Beneficial effects

The present disclosure provides a cream-form pharmaceutical composition/preparation, in which the active ingredient is dispersed as uniform and homogeneous emulsion droplets, exhibiting favorable physical and chemical stability. The composition is suitable for treating atopic dermatitis and seborrheic dermatitis, particularly pediatric atopic dermatitis and sebopsoriasis, while ensuring good safety and efficacy simultaneously.

### BRIEF DESCRIPTION OF DRAWINGS

FIG. 1 shows the exterior view of Example 1 after placing for one week;
FIG. 2 is a microscopic image of Example 2;
FIG. 3 is a microscopic image of Example 3;
FIG. 4 is a microscopic image of Example 6;
FIG. 5 is a microscopic image of commercially available Benvitimod cream;
FIG. 6 is a polarizing microscopic image of commercially available Benvitimod cream;
FIG. 7 is a comparative chart of the skin retention amounts from in-vitro transdermal tests of Example 6 and Comparative Example (commercially available Benvitimod); in the bar chart of Comparative Example and Example 6, the first, second, third, fourth, fifth, and sixth "bars" from left to right correspond to the results of the 1st, 2nd, 3rd, 4th, 5th, and 6th experiments of Comparative Example or Example 6, respectively;
FIG. 8 is a comparative chart of the skin penetration amounts from in-vitro transdermal tests of Example 6 and commercially available Benvitimod;
FIG. 9 shows the clinical effect of the change in erythema index for the treatment of seborrheic dermatitis in Example 6;
FIG. 10 shows the clinical effect of the change in desquamation index for the treatment of seborrheic dermatitis in Example 6; and
FIG. 11 is a comparative chart of the skin retention amounts from in-vitro transdermal tests of Example 6 and sample C.

### DETAILED DESCRIPTION

The technical solutions of the present disclosure will be further described hereinafter through the specific examples. It is to be understood that the examples described below are intended to illustrate and explain the present disclosure but are not construed to limit the protective scope thereof. Any technology realized based on the aforementioned content should fall within the scope intended to be protected by the present disclosure.

Unless otherwise stated, the raw materials and reagents used in the following examples are commercial products or can be prepared by known methods.

Commercially available Benvitimod cream: produced by Guangdong Zhonghao Pharmaceutical Co., Ltd; trade name: Symbiox^{®}, and the content of Benvitimod: 1%.

### Example 1 Preliminary Formulation

By comparing the formulation disclosed in the product instructions of commercially available Benvitimod cream, which consists of an aqueous phase, an oil phase, an emulsifier, a solvent, and a preservative. The specific formulation is shown in Table 1-1 below.

**Table 1-1**

| | | **Commercially available Benvitimod cream** |
|---|---|---|
| | Solvent | Propylene glycol |
| | Emulsifier | Glyceryl mono and distearate, polysorbate 80 |
| Excipients | Oil phase | Cetyl alcohol; white petrolatum, light liquid paraffin |
| | Aqueous phase | Pured water |
| | Others | Ethyl hydroxybenzoate |

Referring to Table 1-1, the composition of the preliminary formulation of the present application the means to the prescriptions of Example 1 and Example 2, wherein the prescription of Example 1 is shown in Table 1.

**Table 1**

| **Component** | **Percentage % (w/w)** |
|---|---|
| E-isomer of the compound shown in Formula (I) | 1.00 |
| White petrolatum | 2.00 |
| Stearyl alcohol | 6.00 |
| Pgoxol-7 stearate | 15.00 |
| Light liquid paraffin | 12.00 |
| Butylated hydroxytoluene | 0.10 |
| Ethyl hydroxybenzoate | 0.10 |
| Polysorbate 80 | 1.00 |
| Diethylene glycol monoethyl ether | 2.00 |
| Propylene glycol | 10.00 |
| Disodium edetate | 0.05 |
| Acetic acid | Appropriate amount |
| Sodium acetate | Appropriate amount |
| Purified water | About 50.75 |
| Total amount | 100 |

The preparation method for the cream preparation is as follows:
1. aqueous phase: disodium edetate, sodium acetate, polysorbate 80, and purified water were added into a beaker, and the mixed solution was heated to 70-80°C and stirred to dissolve; and the pH of the mixed solution was adjusted to 5-6 to obtain the aqueous phase;
2. oil phase: light liquid paraffin, white petrolatum, pgoxol-7 stearate, stearyl alcohol, ethyl hydroxybenzoate, and butylated hydroxytoluene were added into a beaker, and the mixed system was heated to 70-80°C and stirred to dissolve, so as to obtain the oil phase;
3. a third phase: diethylene glycol monoethyl ether and propylene glycol were heated to 55-60°C, and the E-isomer of the compound shown in Formula (I) was added and dissolved in the mixed system, so as to obtain the third phase;
4. the aqueous phase was transferred into the oil phase, and the mixed system was subjected to emulsification for 15 min and then cooling to 55-60°C; and
5. the third phase was added into the emulsion system of step 4, and the mixed system was subjected to emulsification for 15 min and then cooling, so as to obtain the cream preparation.

### Examples 2-4 Optimization of preliminary formulation

The prescriptions of Examples 2-4 are shown in Table 2.

**Table 2**

| **No.** | **Example 2** | **Example 3** | **Example 4** |
|---|---|---|---|
| **Component** | **Percentage % (w/w)** | **Percentage % (w/w)** | **Percentage % (w/w)** |
| E-isomer of the compound shown in Formula (I) | 1.00 | 1.00 | 1.00 |
| White petrolatum | 9.00 | 5.00 | 3.00 |
| Cetostearyl alcohol | 8.00 | 8.00 | 8.00 |
| Polyethylene glycol hexadecyl octadecyl ether | 3.50 | 3.50 | 3.50 |
| Light liquid paraffin | 6.00 | 6.00 | 6.00 |
| Polyoxyethylene (54) hydrogenated castor oil | 0.50 | 0.50 | 0.50 |
| Glyceryl mono and distearate | 3.00 | 3.00 | 3.00 |
| Ethyl hydroxybenzoate | 0.10 | 0.10 | 0.10 |
| Butylated hydroxytoluene | 0.10 | 0.10 | 0.10 |
| Diethylene glycol monoethyl ether | 2.00 | 2.00 | 2.00 |
| Propylene glycol | 8.00 | 8.00 | 8.00 |
| Disodium edetate | 0.05 | 0.05 | 0.05 |
| Acetic acid | Appropriate amount | Appropriate amount | Appropriate amount |
| Sodium acetate | Appropriate amount | Appropriate amount | Appropriate amount |
| Purified water | About 58.75 | About 62.75 | About 64.75 |
| Total amount | 100 | 100 | 100 |

The preparation method for the cream preparation is as follows:
1. aqueous phase: disodium edetate, anhydrous sodium acetate, and purified water were added into a beaker, and the mixed solution was heated to 70-80°C and stirred to dissolve; the pH of the mixed solution was adjusted to 5-6 to obtain the aqueous phase;
2. oil phase: light liquid paraffin, white petrolatum, polyethylene glycol hexadecyl octadecyl ether, cetostearyl alcohol, glyceryl mono and distearate, ethyl hydroxybenzoate, polyoxyethylene (54) hydrogenated castor oil, and butylated hydroxytoluene were added into a beaker, and the mixed system was heated to 70-80°C and stirred to dissolve, so as to obtain the oil phase;
3. a third phase: diethylene glycol monoethyl ether and propylene glycol were heated to 55-60°C, and the E-isomer of the compound shown in Formula (I) was added and dissolved in the mixed system, so as to obtain the third phase;
4. the third phase was transferred into the oil phase, and the mixed system was subjected to heat preservation with stirring for 10 min; and
5. the mixture obtained from step 4 was added into the aqueous phase, and the mixed system was subjected to emulsification for 10 min, then slowly cooled to 48-52°C, and subjected to emulsification for another 5 min, and then cooled, so as to obtain the cream preparation.

### Example 5 Study on the physical and chemical properties of Examples 1-4

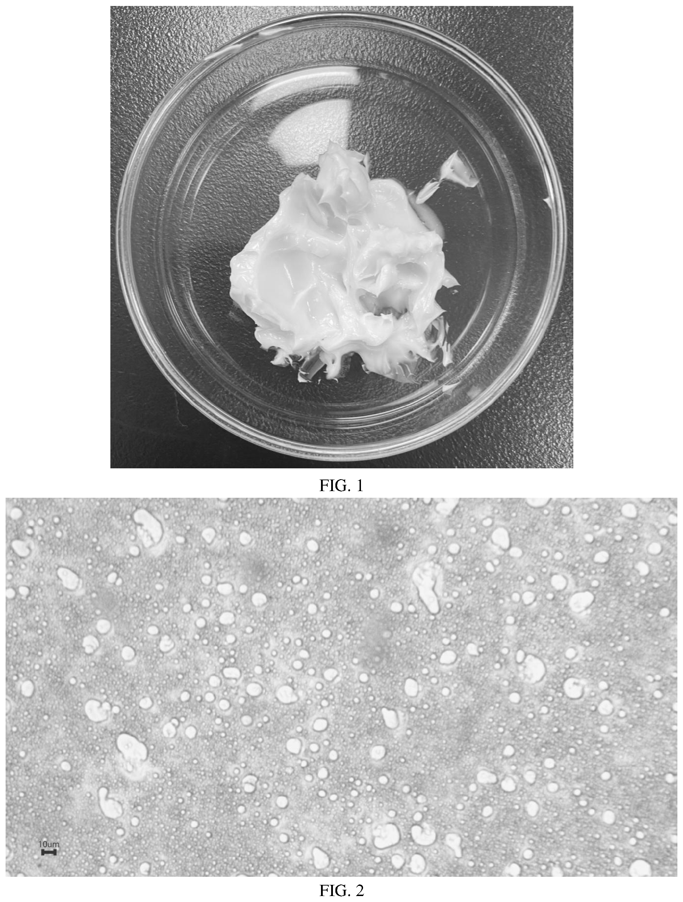

Example 1: off-white cream, which showed uniform emulsion droplets under the microscope, but the cream has a poor cream application sensation and a hard texture; after being placed under conditions of 30°C/RH65% for one week, the appearance of the cream changed significantly, from off-white to pale yellow, as shown in FIG. 1.

Example 2: off-white cream, which showed large oily substances and uneven emulsion droplets under the microscope, and has a poor cream application sensation. The relevant microscopic image is shown in FIG. 2.

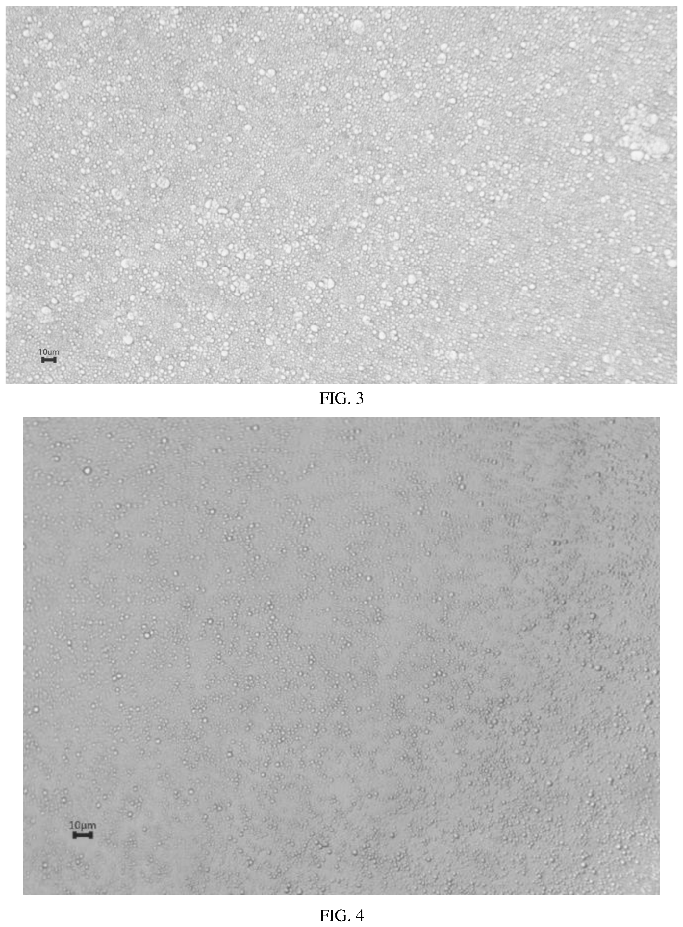

Example 3 and Example 4: by comparing Examples 2-4, the effect of white petrolatum in the prescription on the emulsion droplets of the product was investigated; microscopy showed that the particle size of the emulsion droplets in the sample was decreasing as the proportion of white petrolatum decreased. The relevant microscopic image of Example 3 is shown in FIG. 3.

### Examples 5-7 Formulation optimization

According to the results of the physical and chemical properties of Examples 1-4, the applicant considers continuing to optimize the formulation, and after a lot of creative labor, by adjusting the type and content of factors such as oil phase matrix, thickening agent, pH-adjusting agent, etc., and the optimized formulation finally obtained is shown in Table 3.

**Table 3**

| **No.** | **Example 5** | **Example 6** | **Example 7** |
|---|---|---|---|
| **Component** | **Percentage % (w/w)** | **Percentage % (w/w)** | **Percentage % (w/w)** |
| E-isomer of the compound shown in Formula (I) | 1.00 | 1.00 | 4.00 |
| White petrolatum | 2.00 | 2.00 | 2.00 |
| Cetostearyl alcohol | 7.00 | 7.00 | 7.00 |
| Polyethylene glycol hexadecyl octadecyl ether | 3.50 | 3.50 | 3.50 |
| Medium-chain triglycerides | 10.00 | 10.00 | 10.00 |
| Glyceryl mono and distearate | 3.00 | 3.00 | 3.00 |
| Butylated hydroxytoluene | 0.10 | 0.10 | 0.10 |
| Ethyl hydroxybenzoate | 0.15 | 0.15 | 0.15 |
| Polysorbate 80 | 1.50 | 1.50 | 1.50 |
| Diethylene glycol monoethyl ether | \ | 2.00 | 2.00 |
| Propylene glycol | 10.00 | 8.00 | 8.00 |
| Disodium edetate | 0.05 | 0.05 | 0.05 |
| Citric acid | Appropriate amount | Appropriate amount | Appropriate amount |
| Sodium citrate | Appropriate amount | Appropriate amount | Appropriate amount |
| Purified water | About 61.70 | About 61.70 | About 58.70 |
| Total amount | 100 | 100 | 100 |

The preparation method for the cream preparation in Example 5 is as follows:
first step: preparation of aqueous phase; disodium edetate, sodium citrate, and citric acid were dissolved in purified water, and the mixed solution was heated to 70-80°C and heat preserved; the pH of the mixed solution was adjusted to 4.5-6.5 to obtain the aqueous phase;
second step: preparation of oil phase; the E-isomer of the compound shown in Formula (I), white petrolatum, cetostearyl alcohol, polyethylene glycol hexadecyl octadecyl ether, glyceryl mono and distearate, medium-chain triglycerides, butylated hydroxytoluene, polysorbate 80, propylene glycol, and ethyl hydroxybenzoate was heated to melting at 70-80°C and heat preserved, so as to obtain the oil phase; and
third step: the oil phase was transferred into the aqueous phase, and the mixed system was subjected to emulsification for 10 min and then cooled, so as to form the cream.

The preparation method for the cream preparation in Examples 6-7 is as follows:
first step: preparation of aqueous phase; disodium edetate, sodium citrate, and citric acid were dissolved in purified water, and the mixed solution was heated to 70-80°C and heat preserved; the pH of the mixed solution was adjusted to 4.5-6.5 to obtain the aqueous phase;
second step: preparation of oil phase; the E-isomer of the compound shown in Formula (I), white petrolatum, cetostearyl alcohol, polyethylene glycol hexadecyl octadecyl ether, glyceryl mono and distearate, medium-chain triglycerides, butylated hydroxytoluene, polysorbate 80, diethylene glycol monoethyl ether, propylene glycol, and ethyl hydroxybenzoate was heated to melting at 70-80°C and heat preserved; and
third step: the oil phase was transferred into the aqueous phase, and the mixed system was subjected to emulsification for 10 min and then cooled, so as to form the cream.

### Example 8 Morphology test

The cream preparation obtained by Example 6 was observed with a microscope, and it was found that the active ingredient was uniformly dispersed in the form of emulsion droplets, and the particle size of emulsion droplets was uniform and less than 5 µm, and the microscopic image is shown in FIG. 4.

### Example 9 In vitro transdermal test

*In vitro* transdermal test (IVPT) was used as an index to investigate the effect of diethylene glycol monoethyl ether on skin retention amount.

### 1) Experimental steps:

The preparations prepared in Example 5 and Example 6 were subjected to *in vitro* transdermal tests to evaluate the average retention amount of Benvitimod in the epidermis and dermis. Pig skin (with a thickness of 0.8-1.0 mm) was mounted on the LOGAN System 918 vertical diffusion cell, exposing a surface area of 1.77 cm². The diffusion cell was connected to a multi-channel pump with a rotational speed of 600 rpm and an accepting liquid of PBS (a phosphate buffer solution) at a pH of 7.4. Each cell was placed in a heating manifold to equilibrate the temperature, ensuring a skin surface temperature of 32°C (at least 30 min before administration). Dispense at a dose of 10 mg test sample per square centimeter. The accepting liquid was collected at 2, 4, 8, 20, 24 h to measure the active ingredient that penetrated the skin. At the end of the 24 h sampling, the skin was wiped with a cotton swab to remove any residual samples considered impermeable to the skin, and the epidermis was separated from the dermis by heating. The skin layer was minced, added with methanol, then subjected to ultrasonic to extract the drug, and the concentration of Benvitimod was detected by high-performance liquid chromatography. The recovered drug concentration was used to calculate the average retention amount of Benvitimod in the skin.

### 2) Test results

In the preparation prepared by Example 5, the average retention amount of Benvitimod in the skin is 1.85 µg/cm², and in the preparation prepared by Example 6, the average retention amount of Benvitimod in the skin is 2.91 µg/cm². The results of the transdermal test showed that the addition of the penetrant enhancer diethylene glycol monoethyl ether could significantly increase the total retention amount in the skin.

### Example 10 Irritation test

60 healthy adult guinea pigs, 30♀/30♂, 40 healthy young guinea pigs, 20♀/20♂, were randomly divided into negative control group, positive control group, adult animal for test - low-dose group (Example 6, 1%), adult animal for test - high-dose group (Example 7, 4%), young animal for test - low-dose group (Example 6, 1%), and young animal for test - high-dose group (Example 7, 4%) according to gender and weight by using the software TOXSTAT2006. There were 10 animals (5♀/5♂) in each of the negative control group and the positive control group, and 20 animals (10♀/10♂) in each group in the for test group.

Hair preparation: before administration, the animal should first cut off the skin coat on both sides of the back spine using an electric hair shearing, and the left and right sides were both 3×3 cm².

Sensitization: on the 0th, 7th and 14th days, respectively, 0.2 g/individual of test object (0.2 mL/ individual of negative/positive control group) was given to the left side of the hair removal area, a cellophane or a filter paper was pasted on the groove of the blank plaster, and the test object was placed on the cellophane or covered in the filter paper and applied to the administration site, and then sealed and fixed with non-irritating medical tape. After about 6 h, the blank plaster or filter paper was removed. The animals in the negative control group were given 0.9% sodium chloride injection, the animals in the positive control group were given 2% 2,4-dinitrochlorobenzene (ultrasonically prepared with 80% ethanol), and the animals in the for test - low-dose group and for test - high-dose group were given the corresponding concentrations of the preparations prepared in Example 6 and Example 7 (the content of Benvitimod was 1% and 4%, respectively).

Provocation: on the 14th day after the last sensitization, the test subjects were given 0.2 g/ individual on the right side of the guinea pig hair removal area in each group (0.2 mL/ individual in the positive control group, 0.2% 2,4-dinitrochlorobenzene, prepared using acetone solution; 0.2 mL/ individual in the negative control group, 0.9% sodium chloride injection), and the test substance was removed after about 6 h.

Observation: the symptoms of allergic reaction at 1 h and 24 h after sensitization and the symptoms of allergic reaction at 24 h and 48 h after stimulation were observed.

The results showed that under the conditions of this test, the Buehler test evaluation results were negative after guinea pigs were repeatedly given the preparation prepared by Example 6 and Example 7, indicating that with the increase of the content of the active ingredient Benvitimod, there was still no sensitization effect on the skin.

### Example 11 Stability tests

The same amounts of the cream from Example 6 were weighed in an aluminum tube (sealed), and a total of 6 groups were arranged. The physicochemical stability of the samples was observed and determined by liquid phase at room temperature and 40°C, respectively, for 0 days, 40°C-5 days, 40°C-10 days, 40°C-30 days, 40°C-3 months, and 40°C-6 months. The specific results are shown in Table 4. The results show that the cream from Example 6 will not undergo oxidation and color change after placing for a long time, and the active ingredient is stable and still uniformly dispersed in the form of emulsion droplets, without crystallization, and the impurity content is almost unchanged, which shows the cream has excellent stability.

Liquid phase determination method for impurities and content: octadecylsilane bonded silica gel was used as the filler; water was used as the mobile phase A, acetonitrile was used as the flow phase B, and gradient elution was performed; elution gradient: 0 min 80% A → 20-30 min 10% A → 32-40 min 80% A; the flow rate was 1.0 mL per minute; the column temperature was 35°C; the detection wavelength was 220 nm; injection volume was 10 µL.

**Table 4**

| Item | Examination conditions | Solution color | pH | Maximum single impurity | Total impurity | Content |
|---|---|---|---|---|---|---|
| | 0 day | Off-white cream | 5.2 | 0.04% | 0.04% | 101.7% |
| | 40°C-5 days | Off-white cream | 5.2 | 0.04% | 0.04% | 101.7% |
| Example 6 | 40°C-10 days | Off-white cream | 5.2 | 0.03% | 0.03% | 101.2% |
| | 40°C-30 days | Off-white cream | 5.2 | 0.04% | 0.04% | 101.5% |
| | 40°C-3 months | Off-white cream | 5.2 | 0.04% | 0.06% | 101.7% |
| | 40°C-6 months | Off-white cream | 5.2 | 0.08% | 0.08% | 101.3% |

### Example 12 Comparison of the preparation prepared by Example 6 and commercially available Benvitimod cream

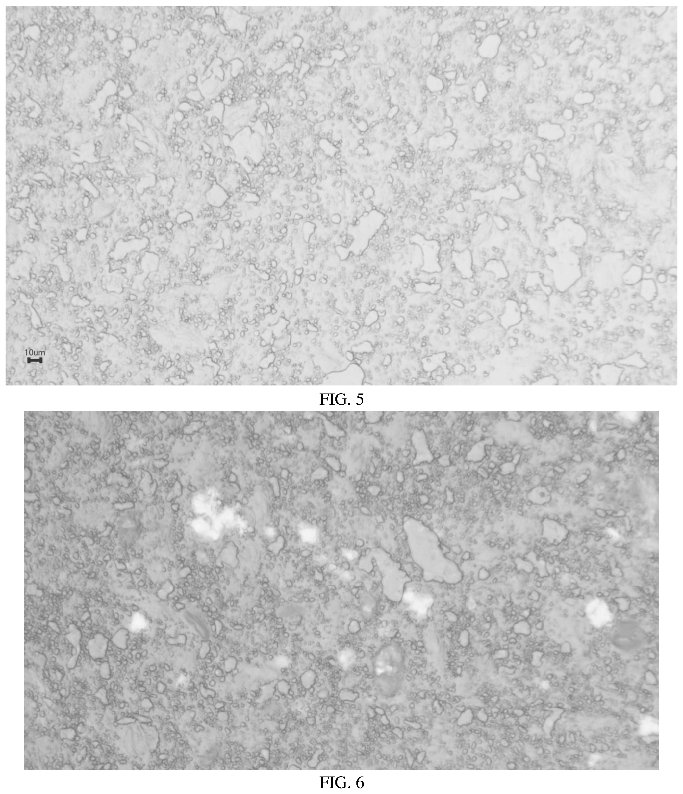

1) Morphology: commercially available Benvitimod cream has uneven emulsion droplets and large oily substances under microscopic conditions (see FIG. 5), and solid crystals were observed under polarized microscopy (see FIG. 6).
*2) In vitro* transdermal test: investigating the retention and penetration amounts of the active ingredient Benvitimod in the skin

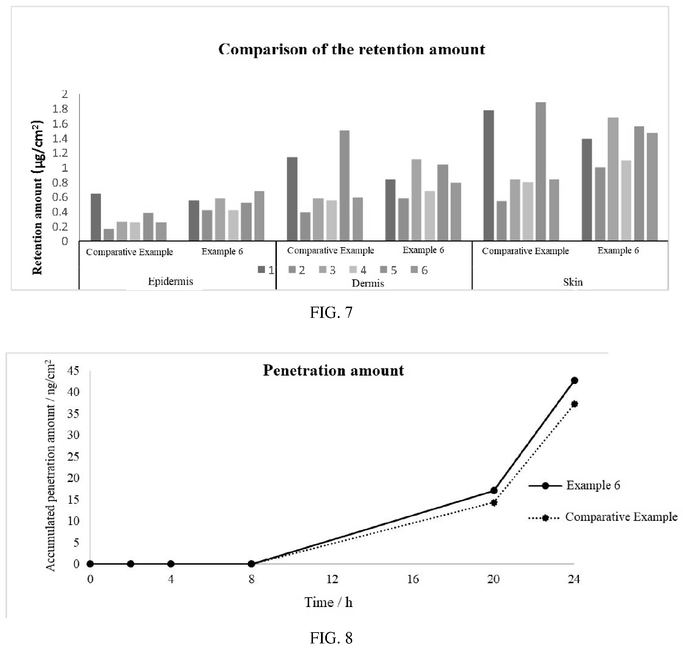

The operations of the *in vitro* transdermal test was carried out with reference to the experimental steps of Example 9, and comparative example and example were tested in parallel for 6 groups (n = 6); the test results are shown in Table 5 and FIGs. 7 and 8.

**Table 5 Retention amount in skin (µg/cm²)**

| | Group | 1 | 2 | 3 | 4 | 5 | 6 | Average value |
|---|---|---|---|---|---|---|---|---|
| Epidermis | Comparative Example | 0.64 | 0.16 | 0.26 | 0.25 | 0.38 | 0.25 | 0.32 |
| | Example 6 | 0.55 | 0.42 | 0.58 | 0.42 | 0.52 | 0.68 | 0.53 |
| Dermis | Comparative Example | 1.14 | 0.39 | 0.58 | *0.55* | 1.51 | 0.59 | 0.79 |
| | Example 6 | 0.84 | 0.58 | 1.11 | 0.68 | 1.04 | 0.79 | 0.84 |
| Skin | Comparative Example | 1.78 | 0.54 | 0.84 | 0.80 | 1.89 | 0.84 | 1.12 |
| | Example 6 | 1.39 | 1.00 | 1.68 | 1.10 | 1.56 | 1.47 | 1.37 |

From Table 5 and the average data of skin retention amount, it can be seen that both Example 6 and comparative example showed that the retention amount of Benvitimod in the dermis was greater than that in the epidermis; in Example 6, the retention amounts of Benvitimod in the dermis and skin were greater than that of the control group. In addition, it can be seen from FIG. 7 that the skin retention amount (dermis layer and epidermis layer) of each group using Example 6 is more uniform and stable than that of each group using comparative example, and the skin retention amount of each group using Example 6 fluctuates less; and from the skin permeability data in FIG. 8, the skin permeability amount of Example 6 is comparable to that of the comparative example, and Example 6 has better parallelism, higher skin retention amount, and better efficacy.

### Example 13

The cream preparation prepared in Example 6 was applied topically to the skin of subjects with atopic dermatitis (AD) and applied to the affected area twice daily, and subjects were aged 3 months - 14 years old. In some patients, significant improvement was observed within 5-7 days, and the symptoms continued to decrease within a month.

### Example 14

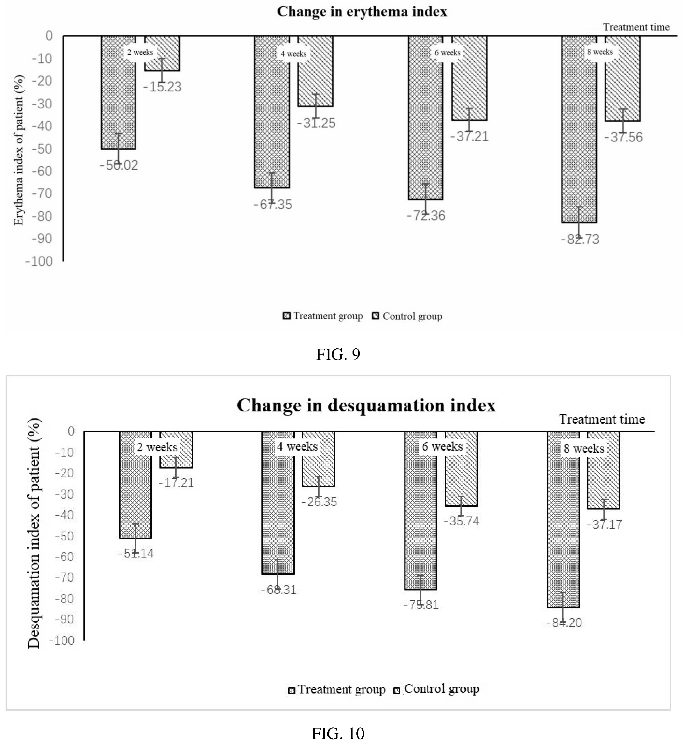

In a prospective, randomized, 8-week clinical trial, the cream preparation prepared in Example 6 (treatment group) was applied to the skin of subjects with seborrheic dermatitis and administered to the affected area twice daily. The control group was administered to the affected area twice daily with the matrix components of Example 6 without Benvitimod. Changes in erythema index and desquamation index in seborrheic dermatitis were significantly improved in the second week and continued into the 8th week. By the 8th week, the change of erythema index and desquamation index in the treatment group decreased by 81.81% and 83.70%. In the control group, the change of erythema index decreased by 38.46%, and the change of desquamation index decreased by 37.44%. Table 6 and FIG. 9 and FIG. 10 show the clinical efficacy of the AhR agonist Benvitimod in the treatment of patients with seborrheic dermatitis.

**Table 6 Clinical efficacy of cream preparation of Example 6 in the treatment of seborrheic dermatitis**

| **Change in erythema index (%)** | | |
|---|---|---|
| Treatment period (weeks) | Treatment group | Control group |
| 2 | -50.02 | -15.23 |
| 4 | -67.35 | -31.25 |
| 6 | -72.36 | -37.21 |
| 8 | -82.73 | -37.56 |

| **Change in desquamation index (%)** | | |
|---|---|---|
| Treatment period (weeks) | Treatment group | Control group |
| 2 | -51.14 | -17.21 |
| 4 | -68.31 | -26.35 |
| 6 | -75.81 | -35.74 |
| 8 | -84.20 | -37.17 |

### Example 15

On the basis of the composition of Example 6, according to the composition of cream preparation samples 1-10 (the composition of sample 9 is the same as Example 6) shown in Table 7 below, and the cream preparation samples 11-19 shown in Table 8 below, the cream preparations were prepared separately, and the effect of the sample composition on the cream preparation product was evaluated.

The preparation method for the cream preparation is as follows:
first step: preparation of aqueous phase; disodium edetate, sodium citrate, and citric acid were dissolved in purified water, and the mixed solution was heated to 70-80°C and heat preserved; the pH of the mixed solution was adjusted to 4.5-5.5 to obtain the aqueous phase;
second step: preparation of oil phase; the E-isomer of the compound shown in Formula (I) was mixed with other components, heated to melting at 70-80°C and heat preserved, so as to obtain the oil phase; and
third step: the oil phase was transferred into the aqueous phase, and the mixed system was subjected to emulsification for 10 min and then cooled, so as to form the cream.

**Table 7**

| **Sample** | **1** | **2** | **3** | **4** | **5** | **6** | **7** | **8** | **9** | **10** |
|---|---|---|---|---|---|---|---|---|---|---|
| **Compone nt** | Percentage % (w/w) | | | | | | | | | |
| E-isomer of the compound shown in Formula (I) | 1.00 | 1.00 | 1.00 | 1.00 | 1.00 | 1.00 | 1.00 | 1.00 | 1.00 | 1.00 |
| White petrolatum | 15.00 | 10.00 | 15.00 | 10.00 | 6.00 | 5.00 | 2.00 | 0.00 | 2.00 | 0.00 |
| Cetostearyl alcohol | 7.00 | 7.00 | 7.00 | 7.00 | 7.00 | 7.00 | 7.00 | 7.00 | 7.00 | 7.00 |
| Medium-c hain triglycerid es | 10.00 | 10.00 | 6.00 | 6.00 | 6.00 | 6.00 | 6.00 | 6.00 | 10.00 | 10.00 |
| Polyethyle ne glycol hexadecyl octadecyl ether | 3.50 | 3.50 | 3.50 | 3.50 | 3.50 | 3.50 | 3.50 | 3.50 | 3.50 | 3.50 |
| Glyceryl mono and distearate | 3.00 | 3.00 | 3.00 | 3.00 | 3.00 | 3.00 | 3.00 | 3.00 | 3.00 | 3.00 |
| Polysorbat e 80 | 1.50 | 1.50 | 1.50 | 1.50 | 1.50 | 1.50 | 1.50 | 1.50 | 1.50 | 1.50 |
| Butylated hydroxytol uene | 0.10 | 0.10 | 0.10 | 0.10 | 0.10 | 0.10 | 0.10 | 0.10 | 0.10 | 0.10 |
| Ethyl hydroxybe nzoate | 0.15 | 0.15 | 0.15 | 0.15 | 0.15 | 0.15 | 0.15 | 0.15 | 0.15 | 0.15 |
| Propylene glycol | 8.00 | 8.00 | 8.00 | 8.00 | 8.00 | 8.00 | 8.00 | 8.00 | 8.00 | 8.00 |
| Diethylene glycol monoethyl ether | 2.00 | 2.00 | 2.00 | 2.00 | 2.00 | 2.00 | 2.00 | 2.00 | 2.00 | 2.00 |
| Disodium edetate | 0.05 | 0.05 | 0.05 | 0.05 | 0.05 | 0.05 | 0.05 | 0.05 | 0.05 | 0.05 |
| Citric acid | Approp riate amount | Approp riate amount | Approp riate amount | Approp riate amount | Approp riate amount | Approp riate amount | Approp riate amount | Approp riate amount | Approp riate amount | Appropr iate amount |
| Sodium citrate | Approp riate amount | Approp riate amount | Approp riate amount | Approp riate amount | Approp riate amount | Approp riate amount | Approp riate amount | Approp riate amount | Approp riate amount | Appropr iate amount |
| Purified water | About 48.70 | About 53.70 | About 52.70 | About 57.70 | About 61.70 | About 62.70 | About 65.70 | About 67.70 | About 61.70 | About 63.70 |
| Total amount | 100 | 100 | 100 | 100 | 100 | 100 | 100 | 100 | 100 | 100 |

**Table 8**

| **Sample** | **11** | **12** | **13** | **14** | **15** | **16** | **17** | **18** | **19** |
|---|---|---|---|---|---|---|---|---|---|
| **Component** | Percentage % (w/w) | | | | | | | | |
| E-isomer of the compound shown in Formula (I) | 1.00 | 1.00 | 1.00 | 1.00 | 1.00 | 1.00 | 1.00 | 1.00 | 1.00 |
| White petrolatum | 3.00 | 3.00 | 3.00 | 3.00 | 3.00 | 3.00 | 3.00 | 3.00 | 3.00 |
| Cetostearyl alcohol | 7.00 | 7.00 | 7.00 | 7.00 | 7.00 | 7.00 | 7.00 | 7.00 | 7.00 |
| Medium-chai n triglycerides | 10.00 | 10.00 | 10.00 | 10.00 | 10.00 | 10.00 | 10.00 | 10.00 | 10.00 |
| Polyethylene glycol hexadecyl octadecyl ether | - | - | 6.00 | 3.50 | 3.50 | 2.00 | 5.00 | 3.50 | 3.50 |
| Glyceryl mono and distearate | 3.00 | 3.00 | 3.00 | 3.00 | 3.00 | 3.00 | 3.00 | - | 6.00 |
| Caprylocapro yl polyoxylglyce rides | 5.00 | - | - | - | - | - | - | - | - |
| Polyoxyethyl ene (54) hydrogenated castor oil | - | 5.00 | - | - | - | - | - | - | - |
| Polysorbate 80 | - | - | - | - | 3.00 | 1.50 | 1.50 | 1.50 | 1.50 |
| Butylated hydroxytolue ne | 0.10 | 0.10 | 0.10 | 0.10 | 0.10 | 0.10 | 0.10 | 0.10 | 0.10 |
| Ethyl hydroxybenzo ate | 0.15 | 0.15 | 0.15 | 0.15 | 0.15 | 0.15 | 0.15 | 0.15 | 0.15 |
| Propylene glycol | 8.00 | 8.00 | 8.00 | 8.00 | 8.00 | 8.00 | 8.00 | 8.00 | 8.00 |
| Diethylene glycol monoethylether | 2.00 | 2.00 | 2.00 | 2.00 | 2.00 | 2.00 | 2.00 | 2.00 | 2.00 |
| Disodium edetate | 0.05 | 0.05 | 0.05 | 0.05 | 0.05 | 0.05 | 0.05 | 0.05 | 0.05 |
| Citric acid | Appropr iate amount | Appropr iate amount | Appropr iate amount | Appropr iate amount | Appropr iate amount | Appropr iate amount | Appropr iate amount | Appropr iate amount | Appropr iate amount |
| Sodium citrate | Appropr iate amount | Appropr iate amount | Appropr iate amount | Appropr iate amount | Appropr iate amount | Appropr iate amount | Appropr iate amount | Appropr iate amount | Appropr iate amount |
| Purified water | About 58.70 | About 60.70 | About 59.70 | About 62.25 | About 59.75 | About 62.25 | About 59.25 | About 63.75 | About 57.75 |
| Total amount | 100 | 100 | 100 | 100 | 100 | 100 | 100 | 100 | 100 |

The results show:
in Table 7, samples 1 and 2 have oily in the upper layers that fail to form a uniform paste. Samples 3-10 can be prepared into cream, but if the amount of white petrolatum decreases, the appearance and color of the cream changes from slightly pink to white cream, and samples 3 and 4 are microscopically observed that no regular emulsion droplets were formed, but appear in small pieces with flaky oil. Samples 5 and 6 were microscopically observed to show that the oil phase could form round emulsion droplets, indicating that if the usage amount of white petrolatum is controlled below 6%, a sample with uniform emulsion droplets could be prepared.

In Table 8, samples 11 and 12 showed large lumpy oil droplets under the microscopic cream, while sample 13 could prepare an off-white cream with small emulsion droplets, indicating that the emulsifier polyethylene glycol hexadecyl octadecyl ether has a good emulsifying ability for the oil phase matrix of medium-chain triglycerides and white petrolatum.

Samples 14-19 can be prepared into a uniform and delicate off-white cream without oil-water stratification. However, the sample 14 without polysorbate 80 had uneven emulsion droplet sizes; the emulsion droplets of sample 15 became smaller and showed round emulsion droplets, indicating that the addition of polysorbate 80 could play a role in improving the emulsion droplets. Sample 18 did not contain glyceryl mono and distearate, had uneven emulsion droplet size and large oil droplets, while this phenomenon was not observed in other samples containing glyceryl mono and distearate.

Furthermore, during the application process, it was observed that samples 13 and 15 contained a high proportion of polysorbate 80 or polyethylene glycol hexadecyl octadecyl ether in the cream, which became whiter and whiter as they were applied. The viscosity test results showed that the viscosity of the cream with a high proportion of polyethylene glycol hexadecyl octadecyl ether and a high proportion of glyceryl mono and distearate is larger than that of the low proportion of cream, indicating polyethylene glycol hexadecyl octadecyl ether and glyceryl mono and distearate have a certain thickening effect.

To sum up, the viscosity of the preferred sample of the present disclosure is moderate, uniform and delicate, and has a very good smearing effect.

### Example 16

On the basis of the composition of the cream preparation in Example 7, according to the composition of the cream preparation samples 20-22 shown in the following Table 9, the cream was prepared separately, and the effect of the active ingredient content in the sample on the cream preparation product was evaluated, wherein the difference between the active ingredient content in the samples 20-22 and Example 7 was adjusted by the purified water content, so that the content of other components remained unchanged.

**Table 9**

| **No.** | **Sample 20** | **Sample 21** | **Sample 22** | **Example 7** |
|---|---|---|---|---|
| Component | Percentage % (w/w) | | | |
| E-isomer of the compound shown in Formula (I) | 0.5 | 2.00 | 3.00 | 4.00 |
| White petrolatum | 2.00 | 2.00 | 2.00 | 2.00 |
| Cetostearyl alcohol | 7.00 | 7.00 | 7.00 | 7.00 |
| Polyethylene glycol hexadecyl octadecyl ether | 3.50 | 3.50 | 3.50 | 3.50 |
| Medium-chain triglycerides | 10.00 | 10.00 | 10.00 | 10.00 |
| Glyceryl mono and distearate | 3.00 | 3.00 | 3.00 | 3.00 |
| Butylated hydroxytoluene | 0.10 | 0.10 | 0.10 | 0.10 |
| Ethyl hydroxybenzoate | 0.15 | 0.15 | 0.15 | 0.15 |
| Polysorbate 80 | 1.50 | 1.50 | 1.5 | 1.50 |
| Propylene glycol | 8.00 | 8.00 | 8.00 | 8.00 |
| Diethylene glycol monoethyl ether | 2.00 | 2.00 | 2.00 | 2.00 |
| Disodium edetate | 0.05 | 0.05 | 0.05 | 0.05 |
| Citric acid | Appropriate amount | Appropriate amount | Appropriate amount | Appropriate amount |
| Sodium citrate | Appropriate amount | Appropriate amount | Appropriate amount | Appropriate amount |
| Purified water | About 62.20 | About 60.70 | About 59.70 | About 58.70 |
| Total amount | 100 | 100 | 100 | 100 |

The preparation method for the cream preparation is as follows:
first step: preparation of aqueous phase; disodium edetate, sodium citrate, and citric acid were dissolved in purified water, and the mixed solution was heated to 70-80°C and heat preserved; the pH of the mixed solution was adjusted to 4.5-5.5 to obtain the aqueous phase;
second step: preparation of oil phase; the E-isomer of the compound shown in Formula (I) was mixed with other components, heated to melting at 70-80°C and heat preserved, so as to obtain the oil phase; and
third step: the oil phase was transferred into the aqueous phase, and the mixed system was subjected to emulsification for 10 min and then cooled, so as to form the cream.

The results show that the compositions of samples 20-22 and Example 7 can be prepared to obtain an off-white cream with a diameter of the emulsion droplet of less than 10 µm, indicating that the cream of the present disclosure can be applied to different contents of the active ingredient.

### Example 17

Sample C was prepared with reference to the composition of preparation 21 in Table 8 of the specification of patent CN114042041A, and the composition of the prescription was an oil phase (an active ingredient, propylene glycol, diethylene glycol monoethyl ether, butylated hydroxytoluene, benzoic acid, emulsifying wax, medium-chain triglycerides, polysorbate 80, steareth-2, and steareth-20) and an aqueous phase (disodium edetate, citric acid, sodium citrate, and purified water). The preparation method is as follows: the oil phase components were mixed and heated to 70-80°C, and continuously mixed until the active ingredient was dissolved and appeared to be uniform; the aqueous phase components were mixed and heated to 70-80°C, and continuously mixed until all substances were completely dissolved and the aqueous phase appeared to be uniform; and the oil phase was added to the aqueous phase and mixed at the temperature kept between 70-80°C for 5-10 min, and the mixture was cooled.

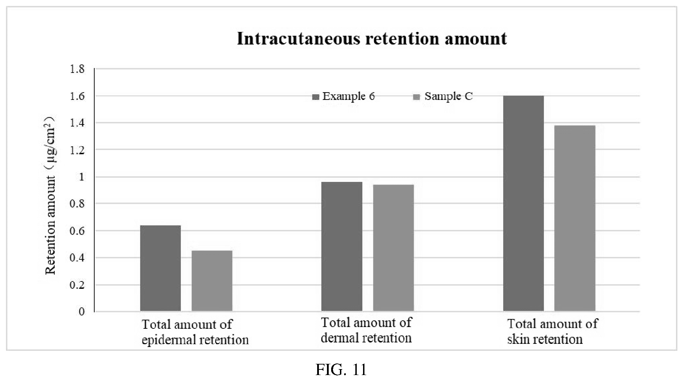

The *in vitro* transdermal test operation referred to the experimental procedure under Experiment 9, and the sample of Example 6 in the present application is compared with sample C, showing that under the condition of the same content of the active ingredient, the intracutaneous retention amount of the cream preparation sample of Example 6 in the present application is higher than that of sample C (see FIG. 11).

The specific embodiments of the present disclosure are illustrated through the examples above. However, the protective scope of the present disclosure is not limited to the above exemplary embodiments. Any modification, equivalent replacement, improvement, etc., made by a person skilled in the art within the spirit and principles of the present disclosure shall fall within the protective scope of the claims of the present disclosure.

## Claims

1. A pharmaceutical composition, comprising the following components: an active ingredient, a thickening agent, an emulsifier, a humectant, a chelating agent, a pH-adjusting agent, an oil phase matrix, and water, wherein the active ingredient is selected from at least one of the compound shown in Formula (I) and pharmaceutically acceptable salts thereof; 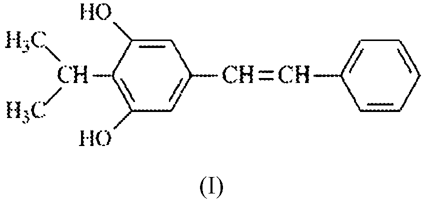
wherein the oil phase matrix comprises petrolatum and optionally existing or non-existing one or two of medium-chain triglycerides and paraffin;
preferably, the oil phase matrix is petrolatum and medium-chain triglycerides;
preferably, in the pharmaceutical composition, petrolatum (preferably white petrolatum) is 1-9 parts by weight, for example, 1-6 parts by weight.

2. The pharmaceutical composition according to claim 1, wherein the compound shown in Formula (I) is an E-isomer or a Z-isomer, preferably the E-isomer shown in Formula (I-1): 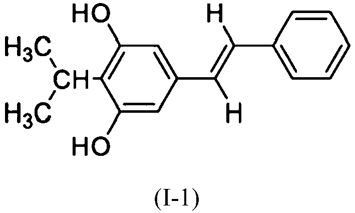
preferably, the pharmaceutically acceptable salt is selected from an additive salt of the compound in Formula (I) and an alkali;
preferably, the pharmaceutical composition uses only the compound shown in Formula (I) as the active ingredient, and preferably only the E-isomer shown in Formula (I-1) as the active ingredient;
preferably, in the pharmaceutical composition, the active ingredient exists in the form of emulsion droplets, preferably a diameter of the emulsion droplet is less than 10 µm;
preferably, in the pharmaceutical composition, the active ingredient is 0.1-8 parts by weight.

3. The pharmaceutical composition according to claim 1 or 2, wherein the thickening agent is selected from one or two of stearyl alcohol and cetostearyl alcohol;
preferably, in the pharmaceutical composition, the thickening agent is 2-15 parts by weight;
preferably, the emulsifier is selected from one or more of polyethylene glycol hexadecyl octadecyl ether, glyceryl mono and distearate, polyethylene glycol stearate, polyoxyethylene hydrogenated castor oil, and polysorbate 80; preferably, the emulsifier comprises at least polyethylene glycol hexadecyl octadecyl ether and glyceryl mono and distearate, for example, the emulsifier is composed of polyethylene glycol hexadecyl octadecyl ether and glyceryl mono and distearate, or composed of polyethylene glycol hexadecyl octadecyl ether, glyceryl mono and distearate, and polysorbate 80;
preferably, in the pharmaceutical composition, the emulsifier is 2-25 parts by weight; if there are two types of emulsifiers, a mass ratio of the two emulsifiers is (1-3):(3-1); if there are three types of emulsifiers, a mass ratio of the three emulsifiers is (1-3):(3-1): 1;
preferably, the humectant is selected from one or more of propylene glycol, glycerol, and polyethylene glycol;
preferably, in the pharmaceutical composition, the humectant is 3-15 parts by weight;
preferably, the chelating agent is selected from a metal-ion chelating agent, for example, selected from one or more of ethylenediaminetetraacetic acid, sodium gluconate, nitrilotriacetic acid, tartaric acid, citric acid and salts thereof;
preferably, in the pharmaceutical composition, the chelating agent is 0.01-1 part by weight;
preferably, the pH-adjusting agent is selected from a pH buffer solution, and preferably a pH buffer solution of an organic acid or a salt thereof; for example, the pH-adjusting agent is selected from one or more of a citric acid/sodium citrate buffer solution, a tartrate buffer solution, a malic acid buffer solution, an acetic acid/sodium acetate buffer solution;
preferably, the oil phase matrix is selected from one or more of petrolatum, medium-chain triglycerides, and paraffin;
preferably, in the pharmaceutical composition, the oil phase matrix is 5-25 parts by weight;
preferably, water is preferably purified water;
preferably, in the pharmaceutical composition, water is 40-65 parts by weight.

4. The pharmaceutical composition according to any one of claims 1-3, wherein the pharmaceutical composition further comprises an antioxidant, a preservative, and/or a penetration enhancer;
preferably, the antioxidant is selected from one or two of propyl gallate and butylated hydroxytoluene;
preferably, in the pharmaceutical composition, the antioxidant is 0.01-0.2 parts by weight;
preferably, the preservative is selected from one or more of methyl hydroxybenzoate, ethyl hydroxybenzoate, and propyl hydroxybenzoate;
preferably, in the pharmaceutical composition, the preservative is 0.01-0.3 parts by weight;
preferably, the penetration enhancer is selected from one or more of diethylene glycol monoethyl ether, isopropyl myristate, isopropyl palmitate, menthol, caprylocaproyl polyoxylglycerides, N-methyl pyrrolidone, propylene carbonate, and isosorbide dimethyl ether;
preferably, in the pharmaceutical composition, the penetration enhancer is 0.1-5 parts by weight; preferably, the pharmaceutical composition has a pH of 4-6.5;
preferably, the pharmaceutical composition further comprises a second active ingredient.

5. The pharmaceutical composition according to any one of claims 1-4, wherein the pharmaceutical composition comprises the following components in parts by weight: 0.1-8 parts of the active ingredient, 2-15 parts of the thickening agent, 2-25 parts of the emulsifier, 3-15 parts of the humectant, 0.01-1 part of the chelating agent, 0.01-0.2 parts of the antioxidant, 0.01-0.3 parts of the preservative, 0-5 parts of the penetration enhancer, 5-25 parts of the oil phase matrix, 40-65 parts of water, and an appropriate amount of the pH-adjusting agent;
the active ingredient is the compound shown in Formula (I), preferably, the E-isomer shown in Formula (I-1) as the active ingredient; and preferably, a pH of the pharmaceutical composition is 4-6.5;
preferably, the pharmaceutical composition comprises the following components in parts by weight:
0.5-5 parts of the E-isomer of the compound shown in Formula (I), 5-10 parts of cetostearyl alcohol, 5-20 parts of the emulsifier, 5-12 parts of propylene glycol, 0.05-0.5 parts of the chelating agent, 0.01-0.2 parts of the antioxidant, 0.01-0.3 parts of the preservative, 0-5 parts of the penetration enhancer, 8-20 parts of the oil phase matrix, 40-65 parts of water, and an appropriate amount of the pH-adjusting agent;
preferably, the emulsifier is composed of polyethylene glycol hexadecyl octadecyl ether, glyceryl mono and distearate, and polysorbate 80 in a mass ratio of (1.5-3):(1.5-3):1;
preferably, the oil phase matrix is composed of white petrolatum and medium-chain triglycerides, wherein the white petrolatum is 1-9 parts.

6. A preparation method for the pharmaceutical composition according to any one of claims 1-5, comprising mixing the components to obtain the pharmaceutical composition.

7. The preparation method according to claim 6, wherein the preparation method is selected from any one of the following methods:
method one, the preparation method comprises the following steps:
(A1) mixing a chelating agent, a pH-adjusting agent, and water, followed by heating to obtain an aqueous phase solution;
(A2) mixing an oil phase matrix, an emulsifier, a thickening agent, and optionally containing or not containing a preservative and an antioxidant, followed by heating to dissolve, to obtain an oil phase solution;
(A3) mixing an active ingredient, a humectant, and optionally containing or not containing a penetration enhancer, followed by heating to dissolve, to obtain a third phase solution;
(A4) transferring the aqueous phase solution to the oil phase solution, emulsifying, and cooling; and
(A5) adding the third phase solution to the emulsion system in step (A4), emulsifying, and cooling, to obtain the pharmaceutical composition;
method two, the preparation method comprises the following steps:
(B1) mixing a chelating agent, a pH-adjusting agent, and water, followed by heating to obtain an aqueous phase solution;
(B2) mixing an oil phase matrix, an emulsifier, a thickening agent, and optionally containing or not containing a preservative and an antioxidant, followed by heating to dissolve, to obtain an oil phase solution;
(B3) mixing an active ingredient, a humectant, and optionally containing or not containing a penetration enhancer, followed by heating to dissolve, to obtain a third phase solution;
(B4) transferring the third phase solution to the oil phase solution, and performing heat preservation and stirring; and
(B5) adding the aqueous phase solution to the mixed system in step (B4), emulsifying, and cooling, and re-emulsifying, and re-cooling, to obtain the pharmaceutical composition;
method three, the preparation method comprises the following steps:
(C1) mixing a chelating agent, a pH-adjusting agent, and water, followed by heating to obtain an aqueous phase solution;
(C2) mixing an active ingredient, an oil phase matrix, an emulsifier, a thickening agent, a humectant, and optionally containing or not containing a preservative, an antioxidant, and a penetration enhancer, followed by heating to dissolve, to obtain an oil phase solution; and
(C3) transferring the oil phase solution to the aqueous phase solution, emulsifying, and cooling, to obtain the pharmaceutical composition.

8. A pharmaceutical preparation, comprising the pharmaceutical composition according to any one of claims 1-5; preferably, the pharmaceutical preparation is a cream preparation.

9. Use of the pharmaceutical composition according to any one of claims 1-5 in the preparation of drugs for treating and/or preventing atopic dermatitis and psoriasis; wherein the atopic dermatitis is preferably pediatric atopic dermatitis, and further preferably, mild, moderate, severe, or refractory pediatric atopic dermatitis.

10. Use of the pharmaceutical composition according to any one of claims 1-5 in the preparation of drugs for treating and/or preventing seborrheic dermatitis; wherein the seborrheic dermatitis is preferably sebopsoriasis.

11. A method for treating and/or preventing atopic dermatitis, psoriasis, and/or seborrheic dermatitis, which comprises administering an effective amount or therapeutic effective amount of the pharmaceutical composition according to any one of claims 1-5 or the pharmaceutical preparation according to claim 8 to a patient; the atopic dermatitis is preferably pediatric atopic dermatitis; the seborrheic dermatitis is preferably sebopsoriasis.
